(19) Europäisches Patentamt
European Patent Office
Office européen des brevets

(11) Veröffentlichungsnummer : **0 125 684**
**B1**

(12) # EUROPÄISCHE PATENTSCHRIFT

(45) Veröffentlichungstag der Patentschrift : **08.07.87**

(51) Int. Cl.⁴ : **C 07 D207/38**, C 07 D401/04, C 07 D401/14, A 61 K 31/40

(21) Anmeldenummer : **84105457.0**

(22) Anmeldetag : **14.05.84**

(54) **2-Alkylthio-1-aminoalkyl-2-pyrrolin-3-carbonitrile, Verfahren zu deren Herstellung sowie diese enthaltende Arzneimittel für die Prophylaxe und Therapie thrombotischer Zustände.**

(30) Priorität : **14.05.83 DE 3317653**

(43) Veröffentlichungstag der Anmeldung : **21.11.84 Patentblatt 84/47**

(45) Bekanntmachung des Hinweises auf die Patenterteilung : **08.07.87 Patentblatt 87/28**

(84) Benannte Vertragsstaaten : **AT BE CH DE FR GB IT LI LU NL SE**

(56) Entgegenhaltungen :
**EP-A- 0 005 156**
**EP-A- 0 091 045**
**CH-A- 313 054**
**AUSTRALIAN JOURNAL OF CHEMISTRY, Band 27, 1974, Seiten 2229-2241; J.W. DUCKER et al.: "The reactions of malononitrile with alpha-diketones and related studies"**

(73) Patentinhaber : **GÖDECKE AKTIENGESELLSCHAFT**
**Salzufer 16**
**D-1000 Berlin 10 (DE)**

(72) Erfinder : **Barth, Hubert, Dr.**
**Rosenweg 60**
**D-7830 Emmendingen (DE)**
Erfinder : **Hartenstein, Johannes, Dr.**
**Fohrenbühl 23**
**D-7801 Stegen-Wittental (DE)**
Erfinder : **Satzinger, Gerhard, Dr.**
**Im Mattenbühl 7**
**D-7809 Denzlingen (DE)**
Erfinder : **Fritschi, Edgar, Dr.**
**Am Scheuerwald 2**
**D-7811 St. Peter (DE)**
Erfinder : **Ganser, Volker, Dr.**
**Sulzburger Strasse 22**
**D-7800 Freiburg (DE)**
Erfinder : **Tauschel, Horst-Dietmar, Dr.**
**Hinterdorfstrasse 29**
**D-7637 Ettenheim (DE)**
Erfinder : **Wolf, Günter, Dr.**
**Wildtalstrasse 40**
**D-7800 Freiburg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Gegenstand der Erfindung sind 2-Alkylthio-1-aminoalkyl-2-pyrrolin-3-carbonitrile der allgemeinen Formel I

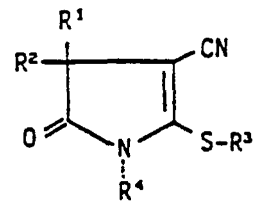

(I)

in welcher $R^1$ und $R^2$ gleich oder verschieden sind und einen Phenyl-, einen Dimethylaminophenyl- oder Pyridylring, $R^3$ eine gerad- oder verzweigtkettige $C_{1-3}$ Alkylgruppe und $R^4$ eine Aminoalkylgruppe der allgemeinen Formel II bedeuten

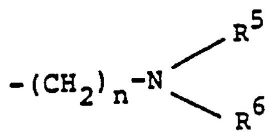

(II)

in der n gleich 2 oder 3 ist, und $R^5$ und $R^6$ gleich oder verschieden sind und Wasserstoff, einen Methyl-, Ethyl-, Propyl- oder Isopropylrest bedeuten oder zusammen mit dem Stickstoffatom, an das sie gebunden sind, eine Pyrrolidino-, Piperidino-, Morpholino- oder Piperazinogruppe darstellen können, sowie deren pharmazeutisch verträgliche Salze.

Die Verbindungen gemäß Formel I werden dadurch hergestellt, daß man 2-Pyrrolin-3-carbonitrile der allgemeinen Formel III

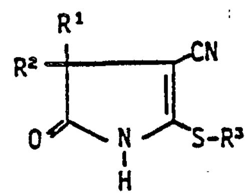

(III)

in der $R^1$, $R^2$ und $R^3$ die obengenannte Bedeutung haben, in einem organischen Lösungsmittel in Gegenwart einer starken Base mit einer Verbindung der allgemeinen Formel IV

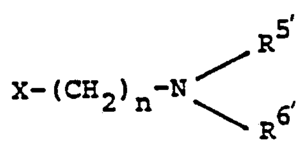

(IV)

in welcher n gleich 2 oder 3 ist und $R^{5'}$ und $R^{6'}$ gleich oder verschieden sind und einen Methyl-, Ethyl-, Propyl- oder Isopropylrest bedeuten oder zusammen mit dem Stickstoffatom, an das sie gebunden sind, eine Pyrrolidino-, Piperidino-, Morpholino- oder Piperazinogruppe darstellen können, wobei einer der Reste $R^{5'}$ und $R^{6'}$ auch Wasserstoff sein kann, und X ein Halogenatom darstellt, umsetzt und die erhaltene Verbindung der allgemeinen Formel I anschließend gegebenenfalls in deren pharmakologisch verträgliche Salze überführt.

Als Lösungsmittel kommen polar protische oder dipolar aprotische Lösungsmittel in Frage. Bevorzugt werden niedere Alkohole, beispielsweise Methanol oder Ethanol, Dioxan oder Tetrahydrofuran. Die Alkylierungsreaktion erfordert die Anwesenheit starker Basen. Bevorzugt werden als Basen beim Arbeiten in polar protischen Lösungsmitteln Natriummethylat und Natriumethylat, beim Arbeiten in dipolar aprotischen Lösungsmitteln Natriumhydrid verwendet. Die Reaktionszeiten betragen zwischen 2 und 24 Stunden.

Die für die Alkylierung verwendeten Alkylierungsmittel der allgemeinen Formel IV werden bevorzugt in Form ihrer Hydrochloride oder -bromide eingesetzt. In diesem Fall verwendet man für die Alkylierung 2 Moläquivalente Base. Bei Verwendung von Dioxan oder Tetrahydrofuran als Lösungsmittel und Natriumhydrid als Base setzt man für die Alkylierung bevorzugt die freie Base der allgemeinen Formel IV ein.

Die Reaktionsprodukte der allgemeinen Formel I kristallisieren nach Filtration vom ausgefallenen Alkalihalogenid direkt aus dem Filtrat aus oder werden nach Einengen der Reaktionsmischung und Verteilen des Rückstands zwischen einem mit Wasser nicht mischbaren organischen Lösungsmittel, beispielsweise Methylenchlorid, und Wasser nach Einengen der organischen Phase und Kristallisieren aus einem geeigneten Lösungsmittel erhalten.

Schwer kristallisierende Basen werden in geeignete Säureadditionssalze überführt. Als solche kommen die Salze organischer und anorganischer Säuren, beispielsweise Salzsäure, Schwefelsäure, Phosphorsäure, Bromwasserstoffsäure, Essigsäure, Maleinsäure, Fumarsäure, Oxalsäure, Bernsteinsäure in Frage. Die Salze werden in an sich bekannter Weise durch Umsetzung der Basen mit entsprechenden organischen und anorganischen Säuren hergestellt.

Ein weiteres Verfahren zur Herstellung von Verbindungen gemäß der Erfindung besteht darin, daß man 2-Pyrrolin-3-carbonitrile der allgemeinen Formel III

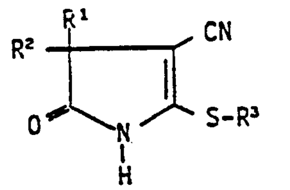

(III)

in der $R^1$, $R^2$ und $R^3$ die obengenannte Bedeutung haben, in einem geeigneten Lösungsmittel in Gegenwart einer starken Base mit einer Verbindung der allgemeinen Formel V

$$X-(CH_2)_n-X' \qquad (V)$$

in welcher n gleich 2 oder 3 ist und X und X' für gleiche oder verschiedene Halogenatome stehen, umsetzt und die erhaltenen Verbindungen der allgemeinen Formel VI

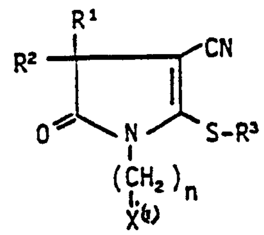

(VI)

in einem geeigneten Lösungsmittel entweder mit einem Alkylamin der allgemeinen Formel VII

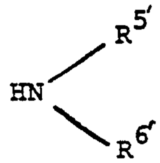

(VII)

in welcher $R^{5'}$ und $R^{6'}$ die obengenannte Bedeutung haben, zu Verbindungen der allgemeinen Formel I umsetzt oder aber indem man eine Verbindung der allgemeinen Formel VIII

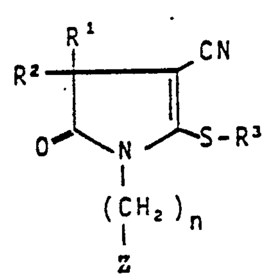

(VIII)

in welcher $R^1$, $R^2$, $R^3$ und n die obengenannte Bedeutung haben und Z den Hexamethylentetrammoniumrest oder eine mit einer sauer abspaltbaren Schutzgruppe versehene Aminogruppe bedeutet, mit einer Säure behandelt.

Als Lösungsmittel für die Herstellung von Verbindungen der allgemeinen Formel VI eignen sich besonders Dioxan oder Tetrahydrofuran. In diesem Falle setzt man Natriumhydrid als Base ein und verwendet das Alkylierungsmittel der allgemeinen Formel V in 10fachem Überschuß. Nach der Reaktion

wird vom ausgefallenen Alkalihalogenid abfiltriert und das Lösungsmittel sowie das überschüssige Alkylierungsmittel im Vakuum entfernt. Der Rückstand wird aus einem geeigneten Lösungsmittel kristallisiert.

Die Umsetzung von Verbindungen der allgemeinen Formel VI mit Aminen der allgemeinen Formel VII wird bevorzugt in Dioxan oder Ethanol in Gegenwart eines Überschusses an Amin bei Raumtemperatur durchgeführt. Die Reaktionszeit beträgt 12 bis 24 Stunden. Nach Entfernen des Lösungsmittels werden die Reaktionsprodukte an Kieselgel chromatographiert.

In den Verbindungen der allgemeinen Formel VIII bedeutet Z den Hexamethylentetrammoniumrest oder eine mit einer sauer abspaltbaren Schutzgruppe versehene Aminogruppe, beispielsweise die tert. Butoxycarbonylaminogruppe, die Benzylidenaminogruppe oder die Triphenylmethylaminogruppe.

Die Herstellung von Verbindungen der allgemeinen Formel IX ist bekannt (vgl. z. B. J. Org. Chem. 48, 24-31, 1983).

Verbindungen, welche das Hexamethylentetrammoniumkation aufweisen erhält man durch Umsetzung von Hexamethylentetramin mit Verbindungen der allgemeinen Formel VI in einem polaren Lösungsmittel, wie z. B. Chloroform.

Die Behandlung der Verbindung der allgemeinen Formel VIII erfolgt bevorzugt unter Verwendung von Mischungen aus konz. Salzsäure und Ethanol bzw. konz. Bromwasserstoffsäure und Ethanol. Nach Entfernen des Säure/Alkoholgemisches im Vakuum wird der Rückstand an Kieselgel chromatographiert und die gewünschte Fraktion nach Einengen kristallisiert. Die als Ausgangsmaterial dienenden Verbindungen der allgemeinen Formel III sind in der DE OS 3212591.7 beschrieben. Die nach dem Verfahren der Erfindung herstellbaren Verbindungen der allgemeinen Formel I sind neu und weisen wertvolle pharmakologische Eigenschaften auf. Insbesondere zeigen sie thrombozytenaggregationsinhibierende Wirkungen. Darüber hinaus sind die erfindungsgemäßen Verbindungen antiphlogistisch, antipyretisch, antisekretorisch und ulkusprotektiv. Die orale Verträglichkeit der Verbindungen ist sehr gut. Ein weiterer Gegenstand der Erfindung ist demgemäß die Verwendung von Verbindungen der allgemeinen Formel I bei der Prophylaxe und Therapie thrombotischer Zustände. Ein weiterer Gegenstand der Erfindung sind daher Arzneimittel, welche mindestens eine der Verbindungen gemäß der allgemeinen Formel I als Wirkstoff enthalten.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z. B. Tartrat- und Citrat-Puffer, Äthanol, Komplexbildner (wie Äthylendiamintetraessigsäure und deren nichttoxische Salze) sowie hochmolekulare Polymere (wie flüssiges Polyäthylenoxid) zur Viskositätsregulierung. Feste Trägerstoffe sind z. B. Stärke, Lactose, Mannit, Methylcellulose, Talkum, hochdisperse Kieselsäuren, höhermolekulare Festtsäuren (wie Stearinsäure), Gelatine, Agar-Agar, Calciumphosphat, Magnesiumstearat, tierische und pflanzliche Fette, feste hochmolekulare Polymere (wie Polyäthylenglykol) ; für orale Applikation geeignete Zubereitungen können gewünschtenfalls zusätzliche Geschmacks- und/oder Süßstoffe enthalten.

Die folgenden Beispiele dienen zur Erläuterung der Erfindung :

## Beispiel 1

2-Ethylthio-5-oxo-4,4-diphenyl-1-(2-piperidinoethyl)-2-pyrrolin-3-carbonitril Hydrochlorid (1)

Man löst 1,38 g Natrium in 200 ml abs. Ethanol, gibt 9,6 g 2-Ethylthio-5-oxo-4,4-diphenyl-2-pyrrolin-3-carbonitril hinzu und erhitzt 30 Min. unter Rückfluß. Danach tropft man innerhalb von 15 Min. eine Lösung von 5,5 g N-(2-Chlor=ethyl)-piperidin Hydrochlorid in 60 ml Ethanol hinzu und kocht 2 Stunden weiter. Die Reaktionsmischung wird eingeengt und der Rückstand zwischen Ether und Wasser verteilt. Die etherische Phase wird über Natriumsulfat getrocknet und schließlich mit etherischer Chlorwasserstofflösung versetzt. Der ausgefallene Niederschlag wird abgesaugt und aus Isopropanol kristallisiert.

Ausbeute 10,5 g farblose Kristalle vom Fp. 210 °C, zers.

In analoger Weise werden erhalten :

1-(2-Diethylaminoethyl)-2-ethylthio-5-oxo-4,4-diphenyl-2-pyrrolin-3-carbonitril Hydrochlorid (2), Ausbeute 73 %, Fp. 187 °C zers.

1-(3-Dimethylaminopropyl)-2-ethylthio-5-oxo-4,4-diphenyl-2-pyrrolin-3-carbonitril Hydrochlorid (3), Ausbeute 46 %, Fp. 188 °C, zers.

1-(3-Dimethylaminoethyl)-2-ethylthio-5-oxo-4,4-diphenyl-2-pyrrolin-3-carbonitril (4), Ausbeute 38 %, Fp. 128 °C

2-Ethylthio-1-(3-morpholinopropyl)-5-oxo-4,4-Diphenyl-2-pyrrolin-3-carbonitril (5), Ausbeute 53 %, Fp. 97 °C

## Beispiel 2

1-(3-Dimethylaminopropyl)-2-isopropylthio-5-oxo-4,4-diphenyl-2-pyrrolin-3-carbonitril (8)

Eine Suspension aus 10,0 g 2-Isopropylthio-5-oxo-4,4-diphenyl-2-pyrrolin-3-carbonitril und 0,9 g Natriumhydrid (80 %) in 150 ml Dioxan wird 1 Stunde unter Rühren gekocht. Danach versetzt man mit weiteren 0,9 g Natriumhydrid und 5,2 g Dimethylaminopropylchlorid Hydrochlorid und kocht 30 Stunden weiter.

Die Reaktionsmischung wird filtriert, das Filtrat zur Trokkene eingeengt und der Rückstand an Kieselgel mit Chloroform/Methanol 10 + 1 chromatographiert. Man erhält 3,7 g farblose Kristalle, Fp. 92 °C

Beispiel 3

1-(2-Diethylaminoethyl)-5-oxo-4,4-diphenyl-2-propylthio-2-pyrrolin-3-carbonitril (9)

Eine Suspension von 10,0 g 5-Oxo-4,4-diphenyl-2-propylthio-2-pyrrolin-3-carbonitril und 0,9 g Natriumhydrid (80 %) in 200 ml Dioxan wird 1 Stunde unter Rückfluß erhitzt. Danach tropft man eine Lösung von 5,8 g Diethylaminoethylchlorid in 20 ml Dioxan hinzu und kocht weitere 6 Stunden am Rückfluß. Die Reaktionsmischung wird filtriert, das Filtrat zur Trockene eingeengt und der Rückstand aus Isopropanol kristallisiert. Ausbeute 8,6 g farblose Kristalle vom Fp. 55 °C.

In analoger Weise werden erhalten :

1-(2-Dimethylaminopropyl)-2-methylthio-5-oxo-4,4-diphenyl-2-pyrrolin-3-carbonitril (6), Ausbeute 76 %, Fp. 106 °C

1-(2-Diethylaminoethyl)-2-methylthio-5-oxo-4,4-diphenyl-2-pyrrolin-3-carbonitril (7), Ausbeute 50 %, Fp. 86 °C

1-(2-Diethylaminoethyl)-2-isopropylthio-5-oxo-4,4-diphenyl-2-pyrrolin-3-carbonitril (10), Ausbeute 56 %, Fp. 66 °C

2-Methylthio-1-(3-morpholinopropyl)-5-oxo-4,4-diphenyl-2-pyrrolin-3-carbonitril (11), Ausbeute 45 %, Fp. 139 °C

2-Methylthio-5-oxo-4,4-diphenyl-1-(3-piperidinopropyl)-2-pyrrolin-3-carbonitril (12), Ausbeute 50 %, Fp. 124 °C

2-Methylthio-5-oxo-4,4-diphenyl-1-(2-pyrrolidinoethyl)-2-pyrrolin-3-carbonitril (13), Ausbeute 43 %, Fp. 97 °C

1-(2-Diisopropylaminoethyl)-2-methylthio-5-oxo-4,4-diphenyl-2-pyrrolin-3-carbonitril (14), Ausbeute 40 %, Fp. 98 °C

2-Ethylthio-5-oxo-4,4-diphenyl-1-(3-piperidinopropyl)-2-pyrrolin-3-carbonitril Oxalat (15), Ausbeute 60 %, Fp. 199 °C zers.

1-(3-Diethylaminopropyl)-2-ethylthio-5-oxo-4,4-diphenyl-2-pyrrolin-3-carbonitril Hydrochlorid (16), Ausbeute 49 %, Fp. 197 °C, zers.

1-(3-Dimethylaminopropyl)-5-oxo-4,4-diphenyl-2-propylthio-2-pyrrolin-3-carbonitril Hydrochlorid (17), Ausbeute 28 %, Fp. 178 °C. Z.

Beispiel 4

2-Ethylthio-5-oxo-1-(2-piperidinoethyl)-4,4-bis(2-pyridyl)-2-pyrrolin-3-carbonitril (18)

Man löst 2,1 g Natrium in 250 ml abs. Ethanol, gibt 14,1 g 2-Ethylthio-5-oxo-4,4-bis(2-pyridyl)-2-pyrrolin-3-carbonitril hinzu und erhitzt 10 Min. unter Rückfluß. Man tropft eine Lösung von 8,3 g N-(2-Chlorethyl)-piperidin Hydrochlorid in 80 ml Ethanol hinzu und kocht 1 Stunde weiter. Die Reaktionsmischung wird eingeengt und der Rückstand zwischen Methylenchlorid und Wasser verteilt. Die organische Phase wird zur Trockene eingeengt und der Rückstand aus Ethanol kristallisiert. Ausbeute 9,9 g farblose Kristalle, Fp. 139 °C In analoger Weise werden erhalten :

1-(3-Dimethylaminopropyl)-2-ethylthio-5-oxo-4,4-bis(2-pyridyl)-2-pyrrolin-3-carbonitril (20), Ausbeute 58 %, Fp. 101 °C

1-(2-Diethylaminoethyl)-2-ethylthio-5-oxo-4,4-bis(2-pyridyl)-2-pyrrolin-3-carbonitril Fumarat (19), Ausbeute 42 %, Fp. 150 °C

Beispiel 5

1-(3-Aminopropyl)-2-ethylthio-5-oxo-4,4-diphenyl-2-pyrrolin-3-carbonitril Hydrochlorid (17a)

Variante A :

Man löst 15,0 g 2-Ethylthio-5-oxo-4,4-diphenyl-2-pyrrolin-3-carbonitril in 500 ml abs. Dioxan, gibt 1,4 g Natriumhydrid (80 % in Weißöl) hinzu und kocht 1 Stunde unter Rückfluß. Man läßt etwas abkühlen, gibt 100,0 g 1,3-Dibrompropan hinzu und kocht 48 Stunden weiter. Danach wird die Reaktionsmischung filtriert, das Lösungsmittel und das überschüssige Dibrompropan werden im Wasserstrahlvakuum abdestilliert und der Rückstand mit Diisopropylether verrieben. Man erhält 14,0 g 1-(3-Brompropyl)-2-ethylthio-5-oxo-4,4-diphenyl-2-pyrrolin-3-carbonitril vom Fp. 97 °C.

8,7 g 1-(3-Brompropyl)-2-ethylthio-5-oxo-4,4-diphenyl-2-pyrrolin-3-carbonitril und 2,8 g Urotropin werden 8 Tage bei Raumtemperatur in 60 ml Chloroform gerührt. Der entstandene Niederschlag wird abfiltriert, mit etwas Chloroform ausgewaschen und getrocknet. Nach Lösen in einer Mischung aus 80 ml Ethanol und 20 ml konz. Salzsäure wird 3 Tage bei Raumtemperatur gerührt. Das Lösungsmittel wird abgezogen und der Rückstand zwischen Chloroform und Wasser verteilt. Die organische Phase wird mit wäßriger Natriumcarbonatlösung ausgewaschen und hiernach mit etherischer Salzsäurelösung versetzt. Nach Entfernen des Lösungsmittels wird der Rückstand an Kieselgel mit Chloroform + Methanol 10 + 1 als Elutionsmittel chromatographiert. Nach Kristallisieren der Hauptfraktion aus Ethanol/Ether erhält man 1,4 g farblose Kristalle vom Fp. 184 °C.

Variante B :

Man löst 16,0 g 2-Ethylthio-5-oxo-4,4-diphenyl-2-pyrrolin-3-carbonitril in 250 ml abs. Dimethylsulfoxid, gibt 1,5 g Natriumhydrid (80 % in Weißöl) hinzu und rührt 15 min. bei Raumtemperatur. Nun tropft man eine Lösung von 13,1 g 3-(tert.-Butoxycarbonylamino)-1-propylbromid in 10 ml abs. Dimethylsulfoxid hinzu, rührt 16 Stunden bei Raumtemperatur und weitere 6 Stunden bei 70 °C. Danach wird das Lösungsmittel im Wasserstrahlvakuum abdestilliert und der Rückstand an Kieselgel mit Chloroform als Elutionsmittel chromatographiert. Nach Kristallisieren der Hauptfraktion aus Essigester/n-Hexan erhält man 10,6 g 1-(3-tert.-Butoxycarbonylaminopropyl)-2-ethylthio-5-oxo-4,4-diphenyl-2-pyrrolin-3-carbonitril vom Fp. 88 °C.

9,5 g 1-(3-tert.-Butoxycarbonylaminopropyl)-2-ethylthio-5-oxo-4,4-diphenyl-2-pyrrolin-3-carbonitril werden in 20 ml mit Salzsäuregas gesättigtem Ethanol suspendiert und ca. 50 Minuten bei Raumtemperatur gerührt. Die zunächst vorliegende Suspension geht langsam in Lösung, danach fällt ein voluminöser Niederschlag aus. Nach Verdünnen mit Diethylether wird der Niederschlag abgesaugt und aus Ethanol kristallisiert. Man erhält 5,7 g 1-(3-Aminopropyl)-2-ethylthio-5-oxo-4,4-diphenyl-2-pyrrolin-3-carbonitril vom Fp. 185 °C

## Beispiel 6

1-(3-Ethylaminopropyl)-2-ethylthio-5-oxo-4,4-diphenyl-2-pyrrolin-3-carbonitril Hydrochlorid (17b)

8,8 g 1-(3-Brompropyl)-2-ethylthio-5-oxo-4,4-diphenyl-2-pyrrolin-3-carbonitril und 3,0 g Ethylamin werden 2 Tage bei Raumtemperatur in 300 ml Dioxan gerührt. Das Lösungsmittel wird am Rotavapor entfernt und der Rückstand zwischen Chloroform und verd. Natriumbicarbonatlösung verteilt. Die organische Phase wird eingeengt und der Rückstand an Kieselgel mit Toluol / Ethanol 10+1 als Elutionsmittel chromatographiert. Die so erhaltene Base wird in ethanolischer Lösung durch Versetzen mit etherischer Salzsäurelösung in das entsprechende Hydrochlorid überführt. Man erhält 1,0 g farblose Kristalle vom Fp. 173-174 °C, zers.

## Beispiel 7

4-(4-Dimethylaminophenyl)-1-(3-dimethylaminopropyl)-2-methylthio-5-oxo-4-(3-pyridyl)-2-pyrrolin-3-carbonitril (21)

Eine Suspension von 3,5 g 4-(4-Dimethylaminophenyl)-2-methylthio-5-oxo-4-(3-pyridyl)-2-pyrrolin-3-carbonitril und 0,3 g Natriumhydrid (80 % in Weißöl) wird eine Stunde rückflussiert. Dann tropft man innerhalb von 10 Min. eine Lösung von 1,3 g 3-Dimethylaminopropylchlorid in 25 ml abs. Dioxan hinzu und kocht 6 Stunden weiter. Nach dem Abkühlen wird die Reaktionsmischung filtriert, das Lösungmittel am Rotavapor entfernt und der Rückstand an Kieselgel mit Chloroform + Methanol 10 + 1 als Elutionsmittel chromatographiert. Nach Kristallisieren der Hauptfraktion aus Isopropanol erhält man 1,8 g farblose Kristalle vom Fp. 90-91 °C.

Untersuchung der akuten Toxizität

Methode :

Die Bestimmung der akuten Toxizität wurde an männlichen Mäusen (NMRI) mit einem Körpergewicht von 18 bis 23 g durchgeführt. Alle Versuchstiere wurden 20 Stunden vor Versuchsbeginn nüchtern gesetzt. Wasser stand ad libitum zur Verfügung. Zu jeder Dosierungsgruppe gehörten 4 Tiere. Die Dosenfolge war logarithmisch. Die Prüfsubstanzen wurden entweder in Form wässriger Lösungen oder als Suspensionen in 0,8 %igem Methocel verabreicht. Das Applikationsvolumen betrug bei subcutaner Applikation 10 ml/kg Körpergewicht, bei intragastraler Verabreichung 20 ml/kg Körpergewicht. Die Tiere wurden insgesamt 7 Tage beobachtet.

Ergebnisse :

Akute Toxizität an Mäusen 7-Tage-Werte

| Verbindungs-Nr. | Applikationsart | $LD_{50}$ mg/kg |
|---|---|---|
| 1 | intragastral | 1600 |
| 5 | intragastral | > 1600 |
| 6 | intragastral | 1200 |
| 8 | intragastral | 1400 |
| 12 | intragastral | > 1600 |
| 15 | intragastral | 700 |
| 21 | intragastral | 1200 |

Kollageninduzierte Thrombozytenaggregation in vitro (Ratte)

Methode :

Der Test wurde nach der Methode von BORN (Nature 194, 927-929, 1962) durchgeführt. Als Blutspender dienten männliche Sprague Dawley SIV 50-Ratten mit einem Körpergewicht von 200 g. Die Blutentnahme geschah unter Äthernarkose aus dem retroorbitalen Venenplexus. 9 Teile Blut wurden mit 1 Teil 3,8 % (w/v) Tri-Na-Citrat-Lösung versetzt. Nach niedrigtouriger Zentrifugation des Gemisches bei Raumtemperatur wurde das Plättchen-reiche Plasma (PRP, entsprechend dem Erythrozyten-freien Überstand) abgenommen und auf eine Standard-Konzentration von 400 000/µl eingestellt. Als Meßinstrument diente ein Universalaggregometer (Braun, Melsungen) verbunden mit einem Eppendorf-Photometer 1 100 M (Netheler und Hinz, Hamburg). Im Versuch wurden jeweils 700 µl PRP im Aggregometer 5 min bei 37 °C äquilibriert und sodann mit 58 µl der Testsubstanzlösung bzw. einer 0,9 %igen NaCl-Lösung (entsprechend dem Leerwert) versetzt. Nach einer Inkubationszeit von 3 min löste die Zugabe von 35 µl Kollagensuspension (Kollagenreagenz HORM$^R$, Hormon Chemie, München) die Aggregation aus. Ein Kompensationsschreiber registrierte in den folgenden 15 min die Änderung der Transmission.

Die größte in diesem Zeitraum auftretende Änderung der Transmission wurde als Amplitude (Amp) der Aggregationskurve bezeichnet. Aggregationshemmende Substanzen bewirken eine Verminderung dieser Amplitude ; die inhibitorische Wirkung (E) einer Substanz errechnete sich nach folgender Formel :

$$E = [(Amp_{Leerwert} - Amp_{Testsubst.})/Amp_{Leerwert}] \times 100 \quad (\%)$$

Es wurden Dosis-Wirkungs-Kurven erstellt, anhand derer $C_{50}$-Werte errechnet werden konnten. Der $C_{50}$-Wert gibt die Endkonzentration einer Substanz im Testsystem an, die zu einer Erniedrigung der Amplitude um 50 % führt.

Ergebnisse :

Kollageninduzierte Thrombozytenaggregation in vitro (Ratte)

| Verbindungs-Nr. | Anzahl der Versuche | $C_{50}$-Werte (mmol/l) Arith. M. ± S.D. |
|---|---|---|
| 3 | 5 | 0,15 ± 0,04 |
| 6 | 5 | 0,19 ± 0,04 |
| 8 | 5 | 0,27 ± 0,05 |
| 12 | 6 | 0,15 ± 0,02 |
| 16 | 6 | 0,14 ± 0,06 |
| 17a | 6 | 0,06 ± 0,00 |
| 21 | 6 | 0,16 ± 0,02 |

Die in der Tabelle aufgeführten Verbindungen zeigen bemerkenswerte aggregationshemmende Eigenschaften, wobei die Verbindung Nr. 17a der wirksamste Vertreter ist.

Untersuchung der antiphlogistischen Wirkung am Carrageeninödem der Ratte

Methode :

Versuchstiere waren männliche, 20 Stunden nüchtern gesetzte Ratten (SIV 50) mit einem Körpergewicht von 110 bis 160 g. Wasser stand ad libitum zur Verfügung. Nach Ermittlung des Ausgangswertes des Pfotenvolumens der rechten Hinterextremität wurden die Prüfsubstanzen intragastral als Suspension in 0,8 %igem Methocel verabreicht. Pro Dosis wurden 10 Tiere verwendet. Das Applikationsvolumen betrug 20 ml/kg Körpergewicht. Das Pfotenvolumen wurde plethysmometrisch gemessen. 60 Minuten nach intragastraler Verabreichung der Prüfsubstanzen wurde 0,1 ml einer 1 %igen Carrageeninlösung subplantar in die rechte Hinterpfote injiziert.

1 und 3 Stunden nach Injektion des Carrageenins wurde das Pfotenvolumen der ödematösen Pfote gemessen. Die Zunahme des Pfotenvolumens der substanzbehandelten Tiere nach 3 Stunden wurde mit derjenigen bei den Kontrolltieren verglichen und die Hemmung des Pfotenödems durch die Prüfsubstanz in Prozent davon berechnet.

Ergebnisse :

Antiphlogistische Wirkung am Carrageeninödem der Ratte

| Verbindungs-Nr. | Dosis mg/kg intragastral | Hemmung des Pfotenödems in % |
|---|---|---|
| 1 | 125 | 36 |
| 5 | 37,5 / 75 | 22 / 45 |
| 6 | 125 | 36 |
| 15 | 150 | 39 |

Untersuchung der antipyretischen Wirkung am Hefefieber der Ratte

Methode :

Versuchstiere waren männliche, 20 Stunden nüchtern gesetzte Ratten (SIV 50) mit einem Körpergewicht von 115 bis 165 g. Zur Erzeugung einer erhöhten Körpertemperatur erhielten die Tiere 18 Stunden vor Verabreichung der Prüfsubstanzen 20 ml/kg Körpergewicht einer 12 %igen Trockenhefesuspension subcutan in 2-3 Depots injiziert. Eine Kontrollgruppe erhielt das entsprechende Volumen physiologischer NaCl-Lösung subcutan injiziert. Pro Dosis wurden 10 Tiere verwendet. Nach 3 Vormessungen der Körpertemperatur am Versuchstag in 30 minütigen Abständen wurde den mit Hefe behandelten Tieren die Prüfsubstanz intragastral verabreicht. Das Applikationsvolumen betrug 20 ml/kg Körpergewicht. Einer zweiten mit Hefe behandelten Kontrollgruppe sowie der am Vortag mit physiologischer NaCl-Lösung behandelten Kontrollgruppe wurde anstatt Prüfsubstanz das entsprechende Vehikel verabreicht. Die Körpertemperatur der Versuchstiere wurde 30, 60 Minuten und dann jeweils stündlich bis zu insgesamt 5 Stunden nach Substanzverabreichung rektal mittels Thermoelementfühler gemessen.

Ergebnisse : Antipyretische Wirkung am Hefefieber der Ratte

| Substanz | Körpertemperatur °C<br>Minuten nach Applikation | | | | | |
|---|---|---|---|---|---|---|
| | 30 | 60 | 120 | 180 | 240 | 300 |
| NaCl 20 ml/kg s.c.<br>Methocel 20 ml/kg i.g. | 36,9 | 36,7 | 36,7 | 36,6 | 36,6 | 36,7 |
| 12% Hefe 20 ml/kg s.c.<br>Methocel 20 ml/kg i.g. | 38,3 | 38,2 | 38,3 | 38,1 | 38,0 | 37,8 |
| 12% Hefe 20 ml/kg s.c.<br>Gö 4775 250 mg/kg i.g.<br>Verb.-Nr. 6 | 37,0 | 36,0 | 35,2 | 34,7 | 35,1 | 35,6 |
| NaCl 20 ml/kg s.c.<br>Methocel 20 ml/kg i.g. | - | 36,8 | 36,9 | 36,9 | 36,9 | 37,0 |
| 12% Hefe 20 ml/kg s.c.<br>Methocel 20 ml/kg i.g. | - | 38,6 | 38,6 | 38,5 | 38,5 | 38,6 |
| 12% Hefe 20 ml/kg s.c.<br>150 mg/kg i.g.<br>Verb.-Nr. 15 | - | 36,6 | 35,8 | 35,9 | 36,4 | 37,0 |

Die aufgeführten Verbindungen zeigen eine deutliche antipyretische Wirkung, wobei es zu einer Absenkung der Körpertemperatur unter die Werte von Tieren ohne Hefefieber kommt.

Untersuchung der ulcusprotektiven Wirkung am durch Indometazin induzierten Magenulcus der Ratte

Methode :

Versuchstiere waren männliche, 20 Stunden nüchtern gesetzte Ratten (SIV 50) mit einem Körpergewicht von 150 bis 200 g. Wasser stand ad libitum zur Verfügung. Den Tieren wurde simultan 40 mg/kg Indometazin und die zu prüfende Substanz als Suspension in 0,8 %igem Methocel intragastral verabreicht. Die Kontrolltiere erhielten nur die entsprechende Dosis Indometazin. Pro Versuchsgruppe

wurden 10 Tiere verwendet. Das Applikationsvolumen betrug 10 ml/kg Körpergewicht. 5 Stunden nach Applikation wurden die Tiere getötet. Nach Entnahme der Mägen und Eröffnung an der Curvatura major wurden diese mittels einer Stereolupe auf das Vorhandensein von Ulcera untersucht. Die Beurteilung der Ulcera erfolgte nach einem von M. Chaumontet et al. (Arzneim.-Forsch. 28, 2119, 1978) vorgeschlagenen Punkteschema. Hieraus wurde der Ulcusindex berechnet. Der Ulcusindex der mit Indometazin und Prüfsubstanz behandelten Tiere wurde mit demjenigen der nur mit Indometazin behandelten Kontrolltiere verglichen und daraus die Ulcushemmung in Prozent berechnet.

Ergebnisse :

Ulcusprotektive Wirkung am durch Indometazin induzierten Magenulcus der Ratte

| Verb.-Nr. | Dosis mg/kg intragastral | Ulcushemmung in % |
|---|---|---|
| 5 | 100 | 52 |
| 9 | 250 | 29 |
| 15 | 150 | 29 |

Verb.-Nr. 5 ist bezüglich der ulcusprotectiven Wirkung der wirksamste Vertreter und zugleich die Verbindung mit dem günstigsten therapeutischen Quotienten.

**Patentansprüche** (für die Vertragsstaaten : BE, CH, DE, FR, GB, IT, LI, LU, NL, SE)

1. 2-Alkylthio-1-aminoalkyl-2-pyrrolin-3-carbonitrile der allgemeinen Formel I

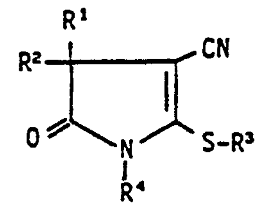

(I)

in welcher $R^1$ und $R^2$ gleich oder verschieden sind und einen Phenyl-, Dimethylaminophenyl- oder Pyridylring, $R^3$ eine gerad- oder verzweigtkettige $C_{1-3}$ Alkylgruppe und $R^4$ eine Aminoalkylgruppe der allgemeinen Formel II bedeuten

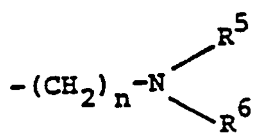

(II)

in der n gleich 2 oder 3 ist, und $R^5$ und $R^6$ gleich oder verschieden sind und Wasserstoff, einen Methyl-, Ethyl-, Propyl- oder Isopropylrest bedeuten oder zusammen mit dem Stickstoffatom, an das sie gebunden sind, eine Pyrrolidino-, Piperidino-, Morpholino- oder Piperazinogruppe darstellen können, sowie deren pharmazeutisch verträgliche Salze.

2. Verfahren zur Herstellung von Verbindungen der allgemeinen Formel I, wobei die Reste $R^1$ bis $R^4$ die in Anspruch 1 genannte Bedeutung haben, dadurch gekennzeichnet, daß man eine Verbindung der allgemeinen Formel III

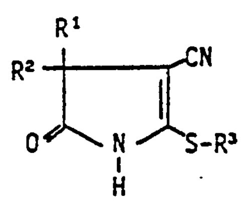

(III)

in welcher $R^1$, $R^2$ und $R^3$ die in Anspruch 1 genannte Bedeutung haben, in einem organischen Lösungsmittel in Gegenwart einer Base mit einem Alkylierungsmittel der allgemeinen Formel IV

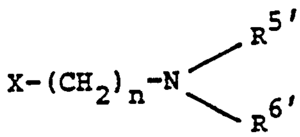

(IV)

in welcher n gleich 2 oder 3 ist und $R^{5'}$ und $R^{6'}$ gleich oder verschieden sind und einen Methyl-, Ethyl-, Propyl- oder Isopropylrest bedeuten oder zusammen mit dem Stickstoffatom, an das sie gebunden sind, eine Pyrrolidino-, Piperidino-, Morpholino- oder Piperazinogruppe darstellen können, wobei einer der Reste $R^{5'}$ und $R^{6'}$ auch Wasserstoff sein kann, und X ein Halogenatom darstellt, umsetzt und die erhaltene Verbindung der allgemeinen Formel I anschließend gegebenenfalls in deren pharmakologisch verträgliche Salze überführt.

3. Verfahren zur Herstellung von Verbindungen der allgemeinen Formel I, wobei die Reste $R^1$ bis $R^4$ die in Anspruch 1 genannte Bedeutung haben dadurch gekennzeichnet, daß man eine Verbindung der allgemeinen Formel III

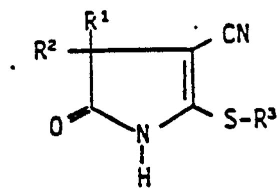

(III)

in welcher $R^1$, $R^2$ und $R^3$ die in Anspruch 1 genannte Bedeutung haben, in einem geeigneten Lösungsmittel in Gegenwart einer geeigneten Base mit einem Alkylierungsmittel der allgemeinen Formel V

$$X-(CH_2)_n-X' \qquad (V)$$

in welcher n gleich 2 oder 3 ist und X und X' für gleiche oder verschiedene Halogen atome stehen, umsetzt und die Verbindungen der allgemeinen Formel VI

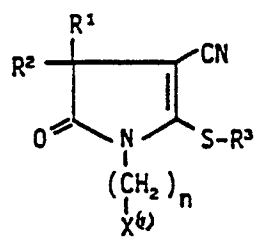

(VI)

in einem geeigneten Lösungsmittel mit einem Alkylamin der allgemeinen Formel VII

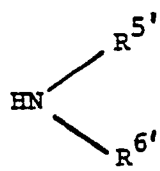

(VII)

in welcher $R^{5'}$ und $R^{6'}$ die in Anspruch 2 genannte Bedeutung haben umsetzt und die erhaltene Verbindung der allgemeinen Formel I gegebenenfalls in deren pharmakologisch verträgliche Salze überführt.

4. Verfahren zur Herstellung von Verbindungen der allgemeinen Formel I

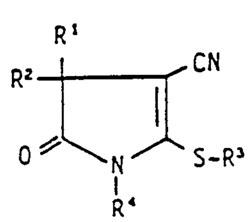

(I)

in welcher $R^1$, $R^2$, $R^3$ und $R^4$ die in Anspruch 1 genannte Bedeutung haben, wobei $R^5$ und $R^6$ Wasserstoff bedeuten, dadurch gekennzeichnet, daß man eine Verbindung der allgemeinen Formel VIII

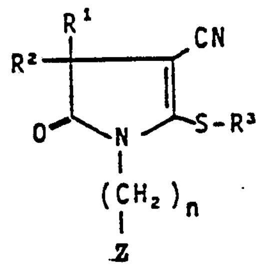

(VIII)

in welcher $R^1$, $R^2$, $R^3$ und n die in Anspruch 1 angegebene Bedeutung haben und Z den Hexamethylen Tetrammoniumrest oder eine mit einer sauer abspaltbaren Schutzgruppe versehene Aminogruppe bedeutet, mit einer Säure behandelt.

5. Verfahren nach Anspruch 2 und 3, dadurch gekennzeichnet, daß man ein polar protisches Lösungsmittel oder aber ein dipolar aprotisches Lösungsmittel verwendet.

6. Verfahren nach Anspruch 5, dadurch gekennzeichnet, daß man als polar protisches Lösungsmittel Methanol oder Ethanol verwendet und daß man als dipolar aprotisches Lösungsmittel Dioxan oder Tetrahydrofuran verwendet.

7. Verfahren nach Ansprüchen 5 und 6, dadurch gekennzeichnet, daß man im Falle der Verwendung eines polar protischen Lösungsmittels Natriummethylat oder Natriumethylat als Base verwendet, und daß man im Falle der Verwendung eines dipolar aprotischen Lösungsmittels Natriumhydrid als Base verwendet.

8. Verfahren gemäß Anspruch 4, dadurch gekennzeichnet, daß man als Gruppe Z den Hexamethylentetrammoniumrest oder die tert. Butoxycarbonylaminogruppe verwendet und zur Umsetzung eine Mischung aus konz. Salzsäure bzw. konz. Bromwasserstoffsäure und Ethanol verwendet.

9. Arzneimittel enthaltend eine Verbindung der allgemeinen Formel I gemäß Anspruch 1.

**Patentansprüche** (für den Vertragsstaat AT)

1. Verfahren zur Herstellung von Verbindungen der allgemeinen Formel I

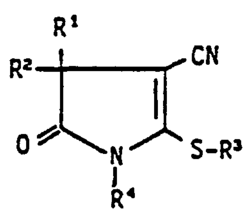

(I)

in welcher $R^1$ und $R^2$ gleich oder verschieden sind und einen Phenyl-, Dimethylaminophenyl- oder Pyridylring, $R^3$ eine gerad- oder verzweigtkettige $C_{1-3}$ Alkylgruppe und $R^4$ eine Aminoalkylgruppe der allgemeinen Formel II bedeuten

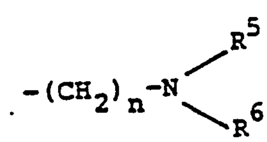

(II)

in der n gleich 2 oder 3 ist, und $R^5$ und $R^6$ gleich oder verschieden sind und Wasserstoff, einen Methyl-, Ethyl-, Propyl- oder Isopropylrest bedeuten oder zusammen mit dem Stickstoffatom, an das sie gebunden sind, eine Pyrrolidino-, Piperidino-, Morpholino- oder Piperazinogruppe darstellen können, dadurch gekennzeichnet, daß man eine Verbindung der allgemeinen Formel III

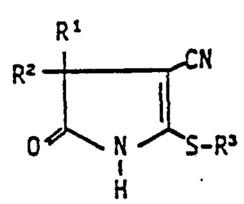

(III)

in welcher $R^1$, $R^2$ und $R^3$ die oben genannte Bedeutung haben, in einem organischen Lösungsmittel in Gegenwart einer starken Base mit einer Verbindung der allgemeinen Formel IV

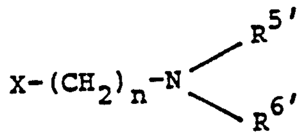

(IV)

in welcher n gleich 2 oder 3 ist und $R^{5'}$ und $R^{6'}$ gleich oder verschieden sind und einen Methyl-, Ethyl-, Propyl-, oder Isopropylrest bedeuten oder zusammen mit dem Stickstoffatom, an das sie gebunden sind, eine Pyrrolidino-, Piperidino-, Morpholino- oder Piperazinogruppe darstellen können, wobei einer der Reste $R^{5'}$ und $R^{6'}$ auch Wasserstoff sein kann, und X ein Halogenatom darstellt, umsetzt und die erhaltene Verbindung der allgemeinen Formel I anschließend gegebenenfalls in deren pharmakologisch verträgliche Salze überführt.

2. Verfahren zur Herstellung von Verbindungen der allgemeinen Formel I, wobei die Reste $R^1$ bis $R^4$ die in Anspruch 1 angegebene Bedeutung haben, dadurch gekennzeichnet, daß man eine Verbindung der allgemeinen Formel III

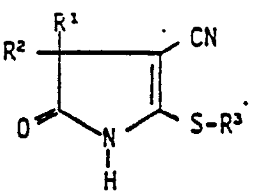

(III)

in welcher $R^1$, $R^2$ und $R^3$ die in Anspruch 1 angegebene Bedeutung haben, in einem geeigneten Lösungsmittel in Gegenwart einer starken Base mit einer Verbindung der allgemeinen Formel V

$$X—(CH_2)_n—X' \qquad (V)$$

in welcher n gleich 2 oder 3 ist und X und X' für gleiche oder verschiedene Halogenatome stehen, umsetzt und die erhaltenen Verbindungen der allgemeinen Formel VI

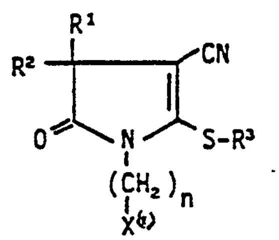

(VI)

in einem geeigneten Lösungsmittel mit einem Alkylamin der allgemeinen Formel VII

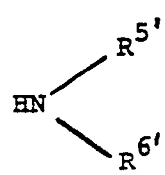

(VII)

in welcher $R^{5'}$ und $R^{6'}$ die in Anspruch 1 angegebene Bedeutung haben, umsetzt und die erhaltene Verbindung der allgemeinen Formel I gegebenenfalls in deren pharmakologisch verträgliche Salze überführt.

3. Verfahren zur Herstellung von Verbindungen der allgemeinen Formel I in welcher $R^1$, $R^2$, $R^3$ und $R^4$ die in Anspruch 1 angegebene Bedeutung haben, wobei jedoch $R^5$ und $R^6$ beide Wasserstoff bedeuten, dadurch gekennzeichnet, daß man eine Verbindung der allgemeinen Formel VIII

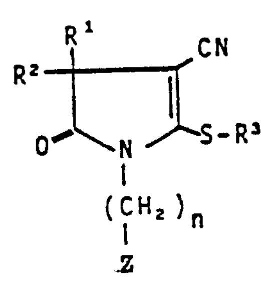

(VIII)

in welcher $R^1$, $R^2$, $R^3$ und n die obengenannte Bedeutung haben und Z eine mit einer sauer abspaltbaren Schutzgruppe versehene Aminogruppe bedeutet, mit einer Säure behandelt.

4. Verfahren nach Anspruch 1 und 2, dadurch gekennzeichnet, daß man ein polar protisches Lösungsmittel oder aber ein dipolar aprotisches Lösungsmittel verwendet.

5. Verfahren nach Anspruch 4, dadurch gekennzeichnet, daß man als polar protisches Lösungsmittel Methanol oder Ethanol verwendet und daß man als dipolar aprotisches Lösungsmittel Dioxan oder Tetrahydrofuran verwendet.

6. Verfahren nach Ansprüchen 4 und 5, dadurch gekennzeichnet, daß man im Falle der Verwendung eines polar protischen Lösungsmittels Natriummethylat oder Natriumethylat als Base verwendet, und daß man im Falle der Verwendung eines dipolar aprotischen Lösungsmittels Natriumhydrid als Base verwendet.

7. Verfahren gemäß Anspruch 3, dadurch gekennzeichnet, daß man als Gruppe Z den Hexamethylentetrammoniumrest oder die tert. Butoxycarbonylaminogruppe verwendet und zur Umsetzung eine Mischung aus konz. Salzsäure bzw. konz. Bromwasserstoffsäure und Ethanol verwendet.

**Claims** (for the Contracting States : BE, CH, DE, FR, GB, IT, LI, LU, NL, SE)

1. 2-Alkylthio-1-aminoalkyl-2-pyrroline-3-carbonitriles of the general formula I

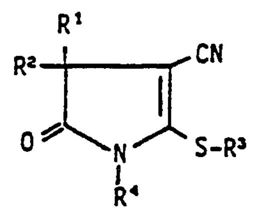

(I)

in which $R^1$ and $R^2$ are the same or different and signify a phenyl-, dimethylaminophenyl- or pyridyl-ring, $R^3$ signifies a straight or branched chain $C_{1\text{-}3}$ alkyl group and $R^4$ signifies an aminoalkyl group of the general formula II

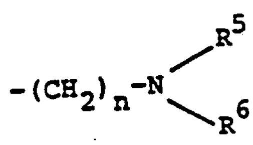

(II)

in which n is equal to 2 or 3 and $R^5$ and $R^6$ are the same or different and individually signify hydrogen, a methyl-, ethyl-, propyl- or isopropyl-group or, together with the nitrogen atom to which they are attached, a pyrrolidino-, piperidino-, morpholino or piperazino group as well as their pharmaceutically acceptable acid addition salts.

2. Process for the preparation of compounds of the general formula I in which the radicals $R^1$ to $R^4$ have the same meaning as in Claim 1, characterized in that one reacts a compound of the general formula III

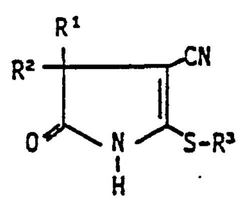

(III)

in which $R^1$, $R^2$, and $R^3$ have the meaning of Claim 1 in an organic solvent in the presence of a base with an alkylation agent of the general formula IV

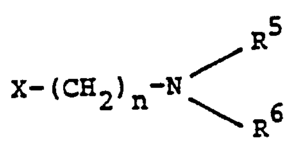

(IV)

in which n equals 2 or 3 and $R^{5'}$ and $R^{6'}$ which may be the same or different represent a methyl-, ethyl-, propyl- or isopropyl radical or together with the nitrogen atom to which they are attached from a pyrrolidino-, piperidino-, morpholino- or piperazino-group and where in one of the radicals $R^{5'}$ and $R^{6'}$ may also be hydrogen, and X stands for a halogen atom, and the compound obtained of the general formula I is possibly converted into its pharmacologically compatible salts.

3. Process for the preparation of compounds of the general formula I, wherem the radicals $R^1$ to $R^4$ have the meaning of claim 1 characterized in that one reacts a compound of the general formula III

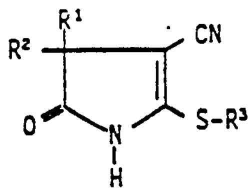

(III)

in which $R^1$, $R^2$, $R^3$ have the meaning of Claim 1 in a suitable solvent in the presence of a suitable base with an alkylation agent of the general formula V

$$X—(CH_2)_n—X' \qquad (V)$$

in which n is 2 or 3 and X and X' which may be the same or different stand for a halogen atom, and the compounds of the general formula VI

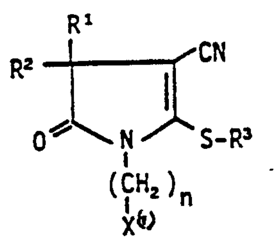

(VI)

are reacted in a suitable solvent with an alkylamine of the general formula VII

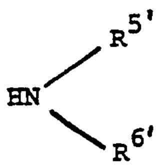

(VII)

wherein $R^{5'}$ and $R^{6'}$ have the meaning of Claim 2, and the compounds obtained of the general formula I are possibly converted into their pharmacologically compatible salts.

4. Process for the preparation of compounds of the general formula I

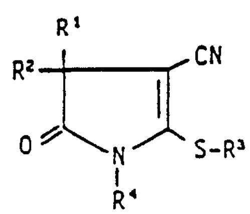

(I)

in which $R^1$, $R^2$, $R^3$ and $R^4$ have the meaning of claim 1, wherein $R^5$ and $R^6$ signify hydrogen, characterized in that one decomposes a compound of the general formula VIII

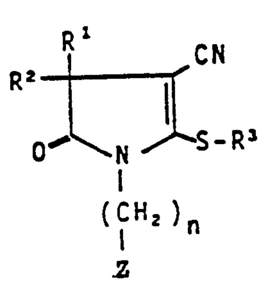

(VIII)

in which $R^1$, $R^2$, $R^3$ and n have the meaning of Claim 1 and Z is a hexamethylenetetrammonium radical or an amino group provided with a protecting group, which may split off by means of an acid, in a per se known manner with an acid.

5. Process according to Claim 2 and 3, characterized in that one uses a polar protic solvent or a dipolar aprotic solvent.

6. Process according to Claim 6, characterized in that, as polar protic solvent, one uses methanol or ethanol and that, as dipolar aprotic solvent, one uses dioxan or tetrahydrofuran.

7. Process according to Claims 5 and 6, characterized in that, in the case of the use of a polar protic solvent, one uses sodium methylate or sodium ethylate as base and, in the case of the use of a dipolar aprotic solvent, one uses sodium hydride as base.

8. Process according to Claim 4, characterized in that as group Z there is used the hexamethylenetetrammonium radical or the tert. butoxycarbonylamino radical and as an acid a mixture of concentrated hydrochloric or hydrobromic acid with ethanol.

9. Pharmaceutical preparation containing a compound of the general formula I according to Claim 1.

**Claims** (for the Contracting State AT)

1. Process for the preparation of compounds of the general formula I

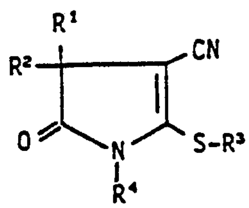

(I)

in which $R^1$ and $R^2$ are the same or different and signify a phenyl-, dimethylaminophenyl- or pyridyl-ring, $R^3$ signifies a straight or branched chain $C_{1-3}$ alkyl group and $R^4$ signifies an aminoalkyl group of the general formula II

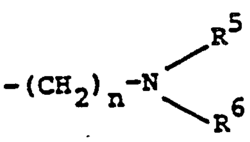

(II)

in which n is equal to 2 or 3 and $R^5$ and $R^6$ are the same or different and signify hydrogen, a methyl-, ethyl-, propyl- or isopropyl-group or, together with the nitrogen atom to which they are attached, can also form a pyrrolidino-, piperidino-, morpholino or piperazino group, characterized in that one reacts a compound of the general formula III

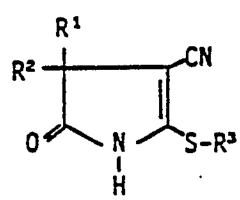

(III)

in which $R^1$, $R^2$, and $R^3$ have the above-mentioned meaning in an organic solvent in the presence of a strong base with a compound of the general formula IV

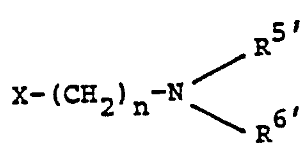

(IV)

in which n equals 2 or 3 and $R^{5'}$ and $R^{6'}$ which may be the same or different represent a methyl-, ethyl-, propyl- or isopropyl group or together with the nitrogen atom to which they are attached form a pyrrolidino-, piperidino-, morpholino- or piperazino-group, and wherein one of the radicals $R^{5'}$ and $R^{6'}$ may also be hydrogen, and X stands for a halogen atom, and the compound obtained of the general formula I is possibly converted into its pharmacologically compatible salts.

2. Process for the preparation of compounds of the general formula I in which the radicals $R^1$ to $R^4$ have the meaning of Claim 1, characterized in that one reacts a compound of the general formula III

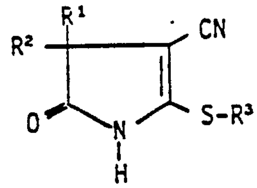

(III)

in which $R^1$, $R^2$, $R^3$ have the meaning of Claim 1 in a suitable solvent in the presence of a strong base with a compound of the general formula V

$$X—(CH_2)_n—X' \qquad (V)$$

in which n equals 2 or 3 and X and X', which may be the same or different, stand for a halogen atom, and the compounds of the general formula VI

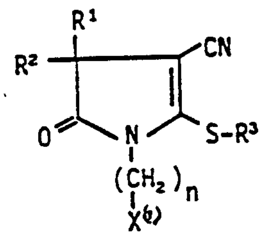

(VI)

are reacted in a suitable solvent with an alkylamine of the general formula VII

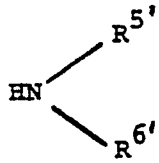

(VII)

wherein $R^{5'}$ and $R^{6'}$ have the meaning of Claim 1, and the compounds obtained of the general formula I are possibly converted into their pharmacologically compatible salts.

3. Process for the preparation of compounds of the general formula I

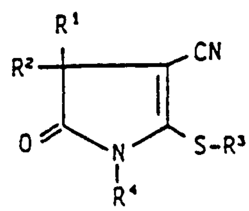

(I)

in which $R^1$, $R^2$, $R^3$ and $R^4$ have the meaning of Claim 1, wherein however both $R^5$ and $R^6$ signify hydrogen, characterized in that one decomposes a compound of the general formula VIII

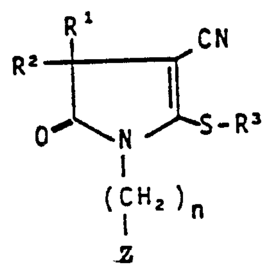

(VIII)

in which $R^1$, $R^2$, $R^3$ and n have the meaning mentioned above and Z is a hexamethylenetetrammonium radical or an amino group provided with a protecting group, which may split off by means of an acid, in a per se known manner with an acid.

4. Process according to Claim 1 and 2, characterized in that one uses a polar protic solvent or a dipolar aprotic solvent.

5. Process according to Claim 4, characterized in that, as polar protic solvent, one uses methanol or ethanol and that, as dipolar aprotic solvent, one uses dioxan or tetrahydrofuran.

6. Process according to Claims 4 and 5, characterized in that, in the case of the use of a polar protic solvent, one uses sodium methylate or sodium ethylate as base and, in the case of the use of a dipolar aprotic solvent, one uses sodium hydride as base.

7. Process according to Claim 3, characterized in that as group Z there is used the hexamethylenetetrammonium radical or the tert. butyloxycarbonylamino radical and as an acid a mixture of concentrated hydrochloric or hydrobromic acid with ethanol.

**Revendications** (pour les Etats contractants : BE, CH, DE, FR, GB, IT, LI, LU, NL, SE)

1. 2-alkylthio-1-aminoalkyl-2-pyrroline-3-carbonitriles de formule générale I :

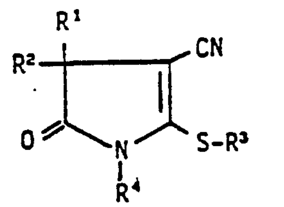

(I)

dans laquelle :

$R^1$ et $R^2$ sont identiques ou différents et représentent un cycle phénylique, diméthylaminophénylique ou pyrridylique ;

$R^3$ représente un groupe alkyle en $C_1$ à $C_3$ à chaîne droite ou ramifiée ; et

$R^4$ représente un groupe aminoalkyle de formule générale II :

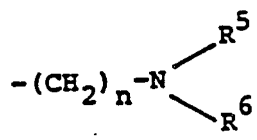

(II)

dans laquelle :

n est égal à 2 ou 3 ; et

$R^5$ et $R^6$ qui sont identiques ou différents, représentent un atome d'hydrogène, un radical méthyle, éthyle, propyle ou isopropyle ou, en même temps que l'atome d'azote auquel ils sont attachés, ils peuvent représenter un groupe pyrrolidino, pipéridino, morpholino ou pipérazino, ainsi que leurs sels pharmaceutiquement acceptables.

2. Procédé de préparation de composés de formule générale I dans lequel les radicaux $R^1$ à $R^4$ cités dans la revendication 1 ont la même signification que ci-dessus, caractérisé en ce que l'on fait réagir un composé de formule générale III :

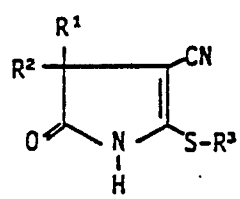

(III)

dans laquelle $R^1$, $R^2$ et $R^3$ ont la signification indiquée dans la revendication 1 ; dans un solvant organique en présence d'une base avec un agent d'alkylation de formule générale IV :

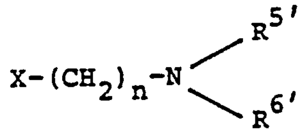

(IV)

dans laquelle :

n est égal à 2 ou 3 ; et

$R^{5'}$ et $R^{6'}$ qui sont identiques ou différents, représentent un radical méthyle, éthyle, propyle ou isopropyle ou, en même temps que l'atome d'azote auquel ils sont attachés, peuvent représenter un groupe pyrrolidino, pipéridino, morpholino ou pipérazino ; l'un des radicaux $R^{5'}$ et $R^{6'}$ pouvant également représenter un atome d'hydrogène ; et

X représente un atome d'halogène, et le cas échéant on fait ensuite passer le composé de formule générale I ainsi obtenu sous la forme de ses sels pharmaceutiquement acceptables.

3. Procédé de préparation de composés de formule générale I, dans lequel les radicaux $R^1$ à $R^4$ ont la signification indiquée dans la revendication 1, caractérisé en ce que l'on fait réagir un composé de formule générale III :

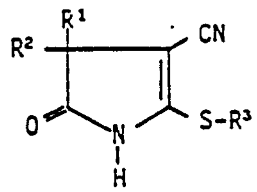

(III)

dans laquelle $R^1$, $R^2$ et $R^3$ ont la signification indiquée dans la revendication 1, dans un solvant approprié, en présence d'une base appropriée, avec un agent d'alkylation de formule générale V :

$$X{-}(CH_2)_n{-}X' \qquad (V)$$

dans laquelle :

n est égal à 2 ou 3 ; et

X et X' représentent des atomes d'halogène, identiques ou différents, et l'on fait réagir les composés obtenus de formule générale VI :

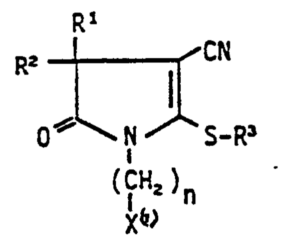

(VI)

dans un solvant approprié avec une alkylamine de formule générale VII :

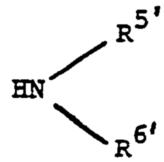

(VII)

dans laquelle $R^{5'}$ et $R^{6'}$ ont la signification indiquée dans la revendication 2 ; et l'on fait le cas échéant passer le composé obtenu de formule générale I sous la forme de ces sels pharmaceutiquement acceptables.

4. Procédé de préparation des composés de formule générale I :

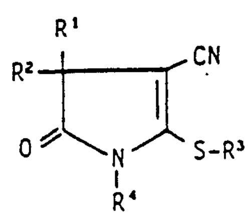

(I)

dans laquelle :

$R^1$, $R^2$, $R^3$ et $R^4$ ont la signification indiquée dans la revendication 1 ;

$R^5$ et $R^6$ représentant un atome d'hydrogène, caractérisé en ce que l'on traite par un acide un composé de formule générale VIII :

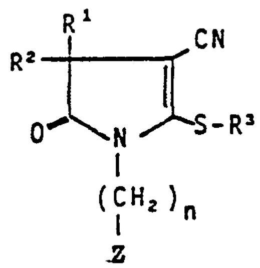

(VIII)

dans laquelle :

$R^1$, $R^2$, $R^3$ et n ont la signification indiquée dans la revendication 1 ;

Z représente un groupe hexaméthylènetetrammonium ou un groupe amino pourvu d'un groupe protecteur séparable par un acide.

5. Procédé selon la revendication 2 et 3, caractérisé en ce que l'on utilise un solvant polaire protique ou un solvant dipolaire aprotique.

6. Procédé selon la revendication 5, caractérisé en ce que l'on utilise comme solvant protique polaire le méthanol ou l'éthanol, et comme solvant dipolaire aprotique le dioxanne ou le tétrahydrofuranne.

7. Procédé selon les revendications 5 et 6, caractérisé en ce que, dans le cas d'utilisation d'un solvant protique polaire, on utilise comme base le méthylate de sodium ou l'éthylate de sodium et que, dans le cas d'utilisation d'un solvant aprotique dipolaire, on utilise comme base l'hydrure de sodium.

8. Procédé selon la revendication 4, caractérisé en ce que l'on utilise comme groupe Z le radical hexaméthylènetetrammonium ou le radical tertiobutoxycarbonylamino et pour la conversion, on utilise un mélange d'acide chlorhydrique concentré ou d'acide bromhydrique concentré et d'éthanol.

9. Médicament contenant un composé de formule générale I selon la revendication 1.

**Revendications** (pour l'Etat contractant AT)

1. Procédé de préparation des composés de formule générale I :

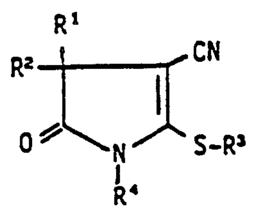

(I)

dans laquelle :

$R^1$ et $R^2$ sont identiques ou différents et représentent un cycle phénylique, diméthylaminophénylique ou pyrridylique ;

$R^3$ représente un groupe alkyle en $C_1$ à $C_3$ à chaîne droite ou ramifiée ; et

$R^4$ représente un groupe aminoalkyle de formule générale II :

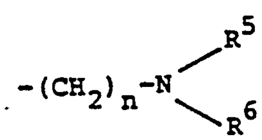

(II)

dans laquelle :

n est égal à 2 ou 3 ; et

$R^5$ et $R^6$ qui sont identiques ou différents, représentent un atome d'hydrogène, un radical méthyle, éthyle, propyle ou isopropyle ou, en même temps que l'atome d'azote auquel ils sont attachés, ils peuvent représenter un groupe pyrrolidino, pipéridino, morpholino ou pipérazino, caractérisé en ce que l'on fait réagir un composé de formule générale III :

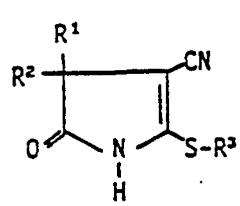

(III)

dans laquelle $R^1$, $R^2$ et $R^3$ ont la signification indiquée ci-dessus, dans un solvant organique en présence d'une base forte avec un composé de formule générale IV :

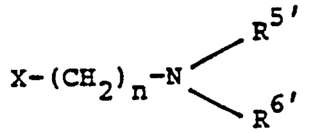

(IV)

dans laquelle :

n est égal à 2 ou 3 ; et

$R^{5'}$ et $R^{6'}$ sont identiques ou différents et représentent un radical méthyle, éthyle, propyle ou isopropyle ou, en même temps que l'atome d'azote auquel ils sont attachés, peuvent représenter un groupe pyrrolidino, pipéridino, morpholino ou pipérazino ; l'un des radicaux $R^{5'}$ et $R^{6'}$ pouvant également représenter un atome d'hydrogène ; et

X représente un atome d'halogène, et le cas échéant, on fait ensuite passer le composé obtenu de formule générale I sous la forme de ses sels pharmaceutiquement acceptables.

2. Procédé de préparation de composés de formule générale I, dans lequel les radicaux $R^1$ à $R^4$ ont la signification indiquée dans la revendication 1, caractérisé en ce que l'on fait réagir un composé de formule générale III :

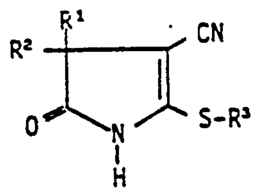

(III)

dans laquelle $R^1$, $R^2$ et $R^3$ ont la signification indiquée dans la revendication 1, dans un solvant approprié, en présence d'une base forte avec un composé de formule générale V :

$$X—(CH_2)_n—X' \quad (V)$$

dans laquelle :

n est égal à 2 ou 3 ; et

X et X′ représentent des atomes d'halogène, identiques ou différents, et l'on fait réagir le composé obtenu de formule générale VI :

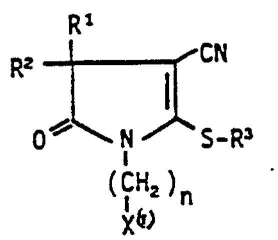

(VI)

dans un solvant approprié avec une alkylamine de formule générale VII :

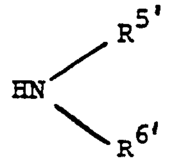

(VII)

dans laquelle $R^{5'}$ et $R^{6'}$ ont la signification indiquée dans la revendication 1 ; et l'on fait, le cas échéant, passer le composé obtenu de formule générale I sous la forme de ces sels pharmaceutiquement acceptables.

3. Procédé de préparation des composés de formule générale I, dans laquelle $R^1$, $R^2$, $R^3$ et $R^4$ ont la signification indiquée dans la revendication 1, $R^5$ et $R^6$ représentant cependant tous les deux un atome d'hydrogène, caractérisé en ce que l'on traite par un acide un composé de formule générale VIII :

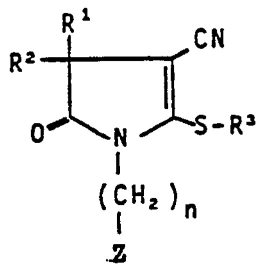

(VIII)

dans laquelle :

$R^1$, $R^2$, $R^3$ et n ont la signification indiquée ci-dessus ; et

Z représente le radical hexaméthylènetetrammonium ou un groupe amino pourvu d'un groupe protecteur séparable par un acide.

4. Procédé selon la revendication 1 ou 2, caractérisé en ce que l'on emploie un solvant polaire protique ou un solvant dipolaire aprotique.

5. Procédé selon la revendication 4, caractérisé en ce que l'on emploie comme solvant protique polaire le méthanol ou l'éthanol, et comme solvant dipolaire aprotique le dioxanne ou le tétrahydrofuranne.

6. Procédé selon les revendications 4 et 5, caractérisé en ce que, dans le cas d'utilisation d'un solvant protique polaire, on emploie le méthylate de sodium ou l'éthylate de sodium comme base et que, dans le cas d'utilisation d'un solvant aprotique dipolaire, on emploie comme base l'hydrure de sodium.

7. Procédé selon la revendication 3, caractérisé en ce que l'on emploie comme groupe Z le radical hexaméthylènetetrammonium ou le radical tertiobutoxycarbonylamino et pour la réaction, on utilise un mélange d'acide chlorhydrique concentré ou d'acide bromhydrique concentré et d'éthanol.